# EUROPÄISCHE PATENTANMELDUNG

(11) **EP 1 767 586 A2**
(43) Veröffentlichungstag der Anmeldung: **28.03.2007**
(21) Anmeldenummer: 06014210.6
(22) Anmeldetag: 08.07.2006
(51) Int. Cl.: C09B 45/14, C09B 67/14

(54) **Verfahren zur Herstellung von Metall-Verbindungen einer Azo-Verbindung unter Anwendung eines mehrstufigen Temperungsverfahren**

(30) Priorität: 19.07.2005 DE 102005033583
(71) Anmelder: LANXESS Deutschland GmbH, 51369 Leverkusen (DE)
(72) Erfinder: Feldhues, Ulrich, Dr., 51465 Bergisch Gladbach (DE); Linke, Frank, 51069 Köln (DE); Göbel, Ronald, 51371 Leverkusen (DE); Endert, Sabine, 42327 Wuppertal (DE); Pfützenreuter, Dirk, 51399 Burscheid (DE)

(57) **Zusammenfassung**

Verfahren zur Herstellung von Metall-Verbindungen einer Azo-Verbindung, die in Form ihrer tautomeren Strukturen der Formel (I) entspricht 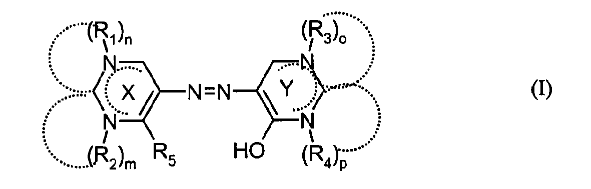 dadurch gekennzeichnet, dass man eine wässrige Suspension einer entsprechenden Metallverbindung in wenigstens zwei pH-Stufen tempert.

## Beschreibung

Die Erfindung betrifft ein Verfahren zur Herstellung von Metall-Verbindungen einer Azo-Verbindung unter Anwendung eines mehrstufigen Temperungsverfahren, die Verwendung der Metallverbindungen als Pigmente und die Verwendung der Pigmente.

Metallkomplexpigmente von Metallen mit einer Azo-Verbindung der folgenden Formel 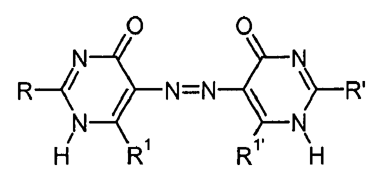 in der
- R und R': unabhängig voneinander für OH, NH₂, NH-CN, Arylamino oder Acylamino
steht und
- R¹ und R^{1'}: unabhängig voneinander -OH oder -NH₂ bedeuten,
sowie Einlagerungsverbindungen daraus sind in der Literatur umfangreich beschrieben, z.B.:
- DE-A-2 064 093
- US-A-4,622,391
- EP 0 994 162 A1
- EP 0 994 163 A1
- EP 0 994 164 A1
- DE 103 28 999 A1.

Es ist auch bekannt, dass die Herstellung durch Tempern erfolgen kann, wobei die Temperung vorteilhaft bei Pigmentkonzentrationen von mehr als 6% erfolgt. Es ist weiterhin bekannt, dass die Temperung unter Zugabe von Festmengen Säuren erfolgen kann. Die nach diesen Methoden hergestellten Pigmente weisen aber noch Nachteile auf, insbesondere bezüglich ihrer Farbstärke.

Aufgabe der vorliegenden Erfindung war es daher, ein kostengünstiges und technisch gut reproduzierbares Verfahren bereitzustellen, das die oben beschriebenen Nachteile nicht mehr aufweist.

Die Erfindung betrifft ein Verfahren zur Herstellung von Metall-Verbindungen einer Azo-Verbindung der Formel (I) 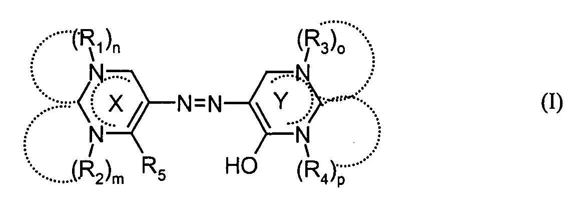 oder tautomere Strukturen davon,
worin
die mit X und Y bezeichneten Ringe je einen oder zwei Substituenten aus der Reihe =O, =S, =NR₇, -NR₆R₇, -OR₆, -SR₆, -COOR₆, -CN, -CONR₆R₇, -SO₂R₈, 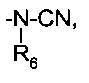 Alkyl, Cycloalkyl, Aryl, Aralkyl tragen können,
wobei die Summe der endo- und exocyclischen Doppelbindungen für jeden der Ringe X und Y drei ist,
worin
- R₆: Wasserstoff, Alkyl, Cycloalkyl, Aryl oder Aralkyl ist,
- R₇: Wasserstoff, Cyano, Alkyl, Cycloalkyl, Aryl, Aralkyl oder Acyl ist und
- R₈: Alkyl, Cycloalkyl, Aryl oder Aralkyl ist,
R₁, R₂, R₃, R₄ für Wasserstoff, Alkyl, Cycloalkyl, Aryl oder Aralkyl stehen und weiterhin, wie in Formel (I) durch die unterbrochenen Linien angedeutet wird, 5- oder 6-gliedrige Ringe ausbilden können, an die weitere Ringe ankondensiert sein können,
- R₅: -OH, -NR₆R₇, Alkyl, Cycloalkyl, Aryl oder Aralkyl bedeutet, worin R₆ und R₇ wie oben definiert sind,
wobei in den für R₁ bis R₈ genannten Substituenten, die CH-Gruppen enthalten, die Wasserstoffatome in den CH-Gruppen substituiert sein können,
und m, n, o, p 1 (eins), oder für den Fall, daß von den Ring-Stickstoffatomen, an denen die entsprechenden Substituenten R, bis R₄ sitzen, Doppelbindungen ausgehen, wie in Formel (I) durch die punktierten Linien angedeutet wird, auch 0 (null) bedeuten können,
und die gegebenenfalls eine Verbindung eingelagert enthalten,
dadurch gekennzeichnet, dass die Metallverbindung oder eine Einlagerungsverbindung davon in wenigstens zwei pH-Stufen getempert wird.

Insbesondere ist das erfindungsgemäße Verfahren dadurch gekennzeichnet, dass man eine wässrige Suspension der Metallverbindung durch Umsetzung einer Azoverbindung der Formel (I) mit einem Metallsalz und gegebenenfalls einer einzulagernden Verbindung in Wasser und gegebenenfalls organischem Lösungsmittel herstellt und die hergestellte Suspension in wenigstens zwei pH-Stufen tempert.

Der Begriff "Tempern" bzw. "Temperung" bedeutet erfindungsgemäß bevorzugt das Halten einer Suspension bzw. Dispersion der Metallverbindung bzw. der Einlagerungsverbindung davon in einem vorzugsweise wässrigen Medium bei einer bestimmten Temperatur und einem bestimmten pH-Wert. Die Temperatur ist in jeder Temperstufe bevorzugt im wesentlichen konstant. So schwankt die Temperatur in einer Temperstufe bevorzugt um nicht mehr als +/- 5° C, bevorzugt um nicht mehr als +/- 3° um einen Mittelwert. Die Temper-Temperatur kann aber auch in jeder Temperstufe schwanken bzw. verändert werden. Entscheidend ist dabei, das bei wenigstens zwei verschiedenen pH-Stufen getempert wird. In den Temper- bzw. pH-Stufen wird bevorzugt ein bestimmter pH-Wert der wässrigen Suspension eingestellt. Der pH-Wert ist dabei in jeder Temperstufe bevorzugt im wesentlichen konstant. Bevorzugt schwankt er in einer Temper- bzw. pH-Stufe um nicht mehr als +/- 1 pH-Einheiten, bevorzugter um nicht mehr als +/- 0,5 pH-Einheiten.

Für den Fachmann ist völlig überraschend, dass durch eine Temperung der Metallverbindung bzw. der Einlagerungsverbindung in wenigstens zwei pH-Stufen gegenüber einer einstufigen Temperung deutlich verbesserte Produkteigenschaften, insbesondere der Farbstärke, und/oder Fertigungskosten erhalten werden können.

Bevorzugt wird die mehrstufige Temperung bevorzugt in einer Dispersion mit einem Feststoffgehalt von mehr als 3 Gew.-%, bevorzugter in einer Dispersion mit einem Feststoffgehalt zwischen 4 - 15 Gew.-%, ganz besonders bevorzugt zwischen 6 - 10 Gew.-% durchgeführt.

Zweckmäßig erfolgt die mehrstufige Temperung in jeder Temperstufe bei Temperaturen zwischen 80 bis 125°C. Bevorzugt wird die mehrstufige Temperung in jeder Temperstufe bei Temperaturen zwischen 90°C und 120°C, insbesondere zwischen 95°C und 110°C durchgeführt. Erfindungsgemäß ist auch der Fall eingeschlossen, dass die Temperatur in jeder Temperstufe im wesentlichen gleich ist. D.h. die Temperung wird bei im wesentlichen gleicher Temperatur aber unterschiedlichen pH-Werten durchgeführt. Die Temperatur kann beispielsweise bei zwei Temperstufen in der ersten Temperstufe niedriger oder höher sein als in der zweiten Temperstufe.

Bevorzugt wird die erfindungsgemäß durchgeführte mehrstufige Temperung in Gegenwart von Wasser und gegebenenfalls organischen Lösungsmitteln bei pH-Werten im Bereich von 0 bis 4 durchgeführt.

Vorzugsweise liegt der pH-Wert von wenigstens einer Temperungsstufe zwischen 2 und 4, insbesondere zwischen 2,5 und 3,5. Vorzugsweise liegt der pH-Wert von einer zweiten Temperungsstufe zwischen 0 und 3, besonders bevorzugt zwischen 1 und 2,5. Vorzugsweise unterscheiden sich die pH-Werte von zwei Temperungsstufen um 0,5 bis 3 Einheiten, vorzugsweise um 1 bis 2 Einheiten. Vorzugsweise wird die zweite von wenigstens zwei Temperungsstufen bei niedrigerem pH-Wert durchgeführt als die erste Temperungsstufe.

Vorzugsweise dauern wenigstens zwei Temperungsstufen unabhängig voneinander zwischen 0,25h und 24h, insbesondere zwischen 1h und 12h, ganz besonders bevorzugt zwischen 2h und 8h.

Vorzugsweise findet die erste Temperstufe bei höherem pH statt als die zweite oder weiteren Temperstufen. Vorzugsweise ist Dauer der ersten Temperstufe kürzer bis maximal gleich lang wie die der zweiten oder weiteren Temperstufen.

Ohne an eine Theorie gebunden zu sein, wird angenommen, dass in der ersten Temperungsstufe, die vorzugsweise bei höheren pH-Werten durchgeführt wird als die zweite bzw. weitere Temperstufe eine Deaggregation der Metallverbindungen bzw. der Einlagerungsverbindungen davon stattfinden, wohingegen in der folgenden Stufe ein Kristallwachstum der Metallverbindungen bzw. der Einlagerungsverbindungen davon stattfindet.

Vorzugsweise wird in der ersten Temperungsstufe des erfindungsgemäßen Verfahrens durch Säurezugabe ein pH insbesondere im Bereich von pH 2,5 bis pH 3,5 eingestellt. In der zweiten bzw. folgenden Temperungsstufe, die sich ggf. auch direkt an die erste Temperungsstufe (insbesondere wenn die zweite Temperungsstufe bei im wesentlichen gleicher Temperatur stattfindet) anschließen kann, wird vorzugsweise über die Zugabe einer Festmenge Säure eine Verringerung des pH-Wertes erreicht. Vorzugsweise sind die nach dem erfindungsgemäßen Verfahren hergestellten Metallverbindungen bzw. Einlagerungsverbindungen davon weitestgehend deaggregiert bzw. deagglomeriert und besitzen eine enge Partikelgrößenverteilung, die insbesondere enger ist als die Partikelgrößenverteilung der eingesetzten Metallverbindungen bzw. Einlagerungsverbindungen davon vor der erfindungsgemäßen Temperung.

Erfindungsgemäß werden unter Metall-Verbindungen einer Azo-Verbindung der Formel (I) insbesondere Metallkomplexverbindungen der Azo-Verbindung der Formel (I) und/oder salzartige Metallverbindungen der Azo-Verbindung der Formel (I) verstanden. Die Azo-Verbindung der Formel (I) liegt in den erfindungsgemäß hergestellten Metall-Verbindungen in der Regel ein- oder mehrfach deprotoniert als Anion vor, wohingegen die Metalle als Kationen vorliegen, die salzartig bis komplexartig bzw. koordinativ (also mit kovalenten Bindungsanteilen) mit dem Anion der Azo-Verbindung der Formel (I) verbunden sind. Die Formel (I) zeigt die Azo-Verbindung in der nicht deprotonierten Form, d.h. in der freien Säureform. Die Herstellung dieser komplexartigen und/oder salzartigen Metallverbindungen beruht vorzugsweise auf der Umsetzung der sauer reagierenden Azoverbindungen der Formel (I) mit Metallverbindungen, wahlweise in Gegenwart von Basen, unter Bildung der Metall-Verbindungen einer Azo-Verbindung der Formel (I).

Die erfindungsgemäß hergestellten Metallverbindungen oder die Einlagerungsverbindungen daraus können auch als Hydrate vorliegen.

Die oben genannte Anzahl der Substituenten an den mit X und Y bezeichneten Ringen (ein oder zwei Substituenten) ist erfindungsgemäß so zu verstehen, dass die eingezeichneten Substituenten R₁ bis R₅ und -OH dabei nicht mitgezählt werden. Die genannten Substituenten an den mit X und Y bezeichneten Ringen sind somit die Substituenten, die an den nicht durch R₁ bis R₅ besetzten Positionen sitzen. Mit den Substituenten R₁ bis R₅ können somit auch mehr als zwei Substituenten an den mit X und Y bezeichneten Ringen sitzen.

In einer bevorzugten Ausführungsform des erfindungsgemäßen Verfahrens steht in der Verbindung der Formel (I) der mit X gekennzeichnete Ring für einen Ring der Formel 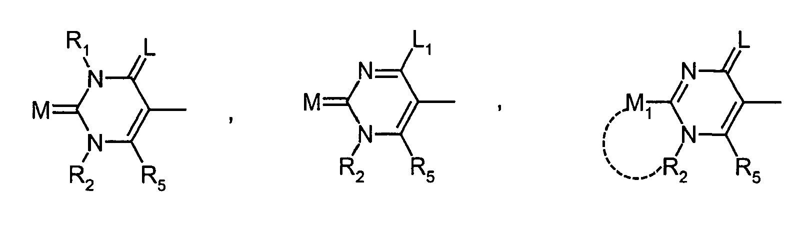 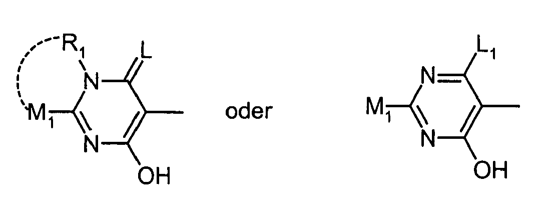 in denen
- L und M: unabhängig voneinander für =O, =S oder =NR₆ stehen,
- L₁: Wasserstoff, -OR₆, -SR₆, -NR₆R₇, -COOR₆, -CONR₆R₇, -CN, Alkyl, Cycloalkyl, Aryl, Aralkyl ist und
- M₁: -OR₆, -SR₆, -NR₆R₇, -COOR₆, -CONR₆R₇, -CN, -SO₂R₈, 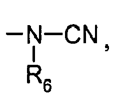 Alkyl, Cycloalkyl, Aryl oder Aralkyl bezeichnen,
oder die Substituenten M₁ und R₁ oder M₁ und R₂ einen 5- oder 6-gliedrigen Ring ausbilden können, und
- R₁, R₂, R₅, R₆, R₇ und R₈: die oben angegebenen Bedeutungen besitzen.

Besonders bevorzugte erfindungsgemäß hergestellte Metallverbindungen sind dabei solche von Azo-Verbindungen, die in Form ihrer freien Säuren Strukturen der Formeln (II) oder (III) entsprechen 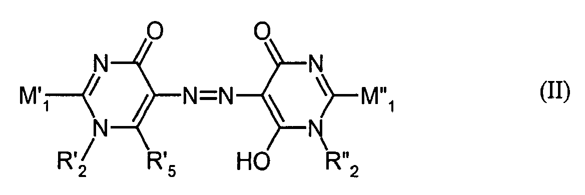 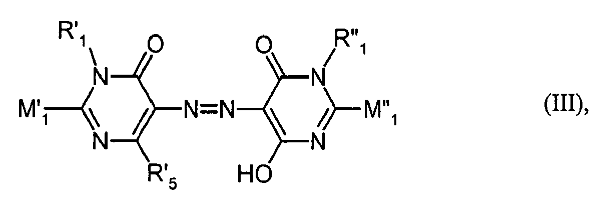 oder dazu tautomeren Formen,
in denen
- R'₅: -OH oder -NH₂ bezeichnet,
- R'₁, R"₁, R'₂ und R"₂: jeweils für Wasserstoff stehen und
- M'₁ und M"₁: unabhängig voneinander für Wasserstoff, -OH, -NH₂, -NHCN, Arylamino oder Acylamino stehen.

Ganz besonders bevorzugte Metallverbindungen sind dabei solche von Azo-Verbindungen der Formel (I), die in Form ihrer freien Säure einer Struktur der Formel (IV) entsprechen 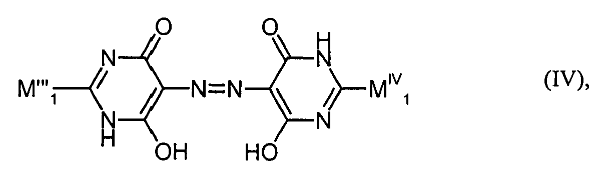 oder tautomeren Strukturen davon,
in denen
- M'''₁ und M^{IV}₁: unabhängig voneinander OH und/oder NHCN bedeuten.

Besonders bevorzugt sind Metallverbindungen von Azo-Verbindungen der Formel: 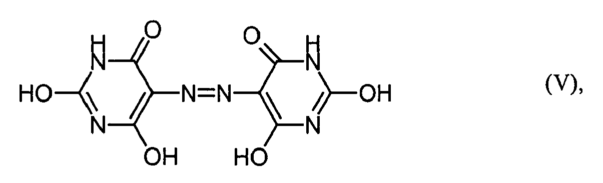 oder dazu tautomeren Strukturen.

In den vorstehenden Formeln haben die Substituenten vorzugsweise die folgenden Bedeutungen:

Substituenten in der Bedeutung von Alkyl bezeichnen vorzugsweise C₁-C₆-Alkyl, das beispielsweise durch Halogen, wie Chlor, Brom, Fluor, -OH, -CN, -NH₂ oder C₁-C₆-Alkoxy substituiert sein kann. Darin bedeutet C₁-C₆-Alkyl geradkettiges oder verzweigtes Alkyl mit 1 bis 6 Kohlenstoffatomen, wie Methyl, Ethyl, Propyl, Butyl, Pentyl , Hexyl einschließlich sämtlicher isomerer Formen davon.

Substituenten in der Bedeutung von Cycloalkyl bezeichnen vorzugsweise C₃-C₇-Cycloalkyl, insbesondere C₅-C₆-Cycloalkyl, das beispielsweise durch C₁-C₆-Alkyl, C₁-C₆-Alkoxy, Halogen wie Cl, Br, F, C₁-C₆-Alkoxy, -OH, -CN und NH₂ substituiert sein kann.

Substituenten in der Bedeutung von Aryl bezeichnen vorzugsweise Phenyl oder Naphthyl, die beispielsweise durch Halogen wie F, Cl, Br, -OH, C₁-C₆-Alkyl, C₁-C₆-Alkoxy, -NH₂, -NO₂ und -CN substituiert sein können.

Substituenten in der Bedeutung von Aralkyl bezeichnen bevorzugt Phenyl- oder Naphthyl-C₁-C₄-alkyl, die in den aromatischen Resten beispielsweise durch Halogen wie F, Cl, Br, -OH, C₁-C₆-Alkyl, C₁-C₆-Alkoxy, -NH₂, -NO₂ und -CN substituiert sein können.

Substituenten in der Bedeutung von Acyl bezeichnen vorzugsweise (C₁-C₆-Alkyl)-carbonyl, Phenylcarbonyl, C₁-C₆-Alkylsulfonyl, Phenylsulfonyl, gegebenenfalls durch C₁-C₆-Alkyl, Phenyl und Naphthyl substituiertes Carbamoyl, gegebenenfalls durch C₁-C₆-Alkyl, Phenyl und Naphthyl substituiertes Sulfamoyl oder gegebenenfalls durch C₁-C₆-Alkyl, Phenyl oder Naphthyl substituiertes Guanyl, wobei die genannten Alkylreste beispielsweise durch Halogen wie Cl, Br, F, -OH, -CN, -NH₂ oder C₁-C₆-Alkoxy substituiert sein können und die genannten Phenyl- und Naphthylreste beispielsweise durch Halogen wie F, Cl, Br, -OH, C₁-C₆-Alkyl, C₁-C₆-Alkoxy, -NH₂, -NO₂ und -CN substituiert sein können.

Für den Fall, dass M₁ zusammen mit R₁ oder M₁ zusammen mit R₂ und/oder R₁, R₂, R₃, R₄, wie in den oben gezeigten Formeln durch die unterbrochenen Linien angedeutet wird, 5- oder 6-gliedrige Ringe ausbilden, handelt es sich vorzugsweise um Triazol-, Imidazol- oder Benzimidazol-, Pyrimidin- oder Chinazolin-Ringsysteme.

Als Metallverbindungen, worunter wie bereits dargelegt, salz- oder komplexartige Metallverbindungen verstanden werden, der Azoverbindungen der Formeln (I) bis (V) kommen vorzugsweise die Salze und Komplexe der Mono-, Di-, Tri- und Tetraanionen der Azoverbindungen der Formeln (I) bis (V) in Betracht. Geeignete Metalle werden zweckmäßig ausgewählt aus einem oder mehreren Metallen, die aus der Gruppe ausgewählt werden, die besteht aus: Li, Na, K, Mg, Ca, Sr, Ba, Al, Sn, Pb, Sc, Ti, V, Cr, Mn, Fe, Co, Ni, Cu, Zn, Y, Zr, Nb, Mo, Cd, Hf, Ta, W, La, Ce, Pr und Nd. Bevorzugt ist Nickel.

Besonders bevorzugt sind Salze und Komplexe der Formeln (I) bis (V) mit zwei- oder dreiwertigen Metallen, ganz besonders die Nickelsalze und -komplexe. In einer bevorzugten Ausführunsgform des erfindungsgemäßen Verfahrens wird ein Ni-Salz bzw. eine Ni-Komplexverbindung der Azoverbindung der Formel (I) hergestellt.

Bevorzugt handelt es sich bei der Metallverbindung um den Azobarbitursäure-Nickel-1:1-Komplexes der Struktur 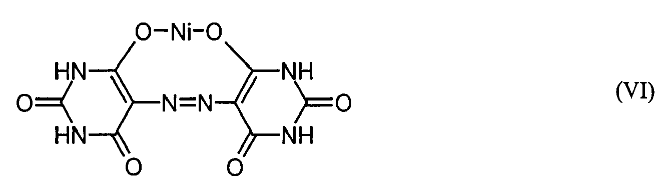 oder eine tautomere Struktur dazu.

Die erfindungsgemäß hergestellten Metallverbindungen können gegebenenfalls eine oder mehrere Verbindungen eingelagert enthalten. Bevorzugt handelt es sich bei der eingelagerten Verbindung um eine organische Verbindung, d.h. um eine solche Verbindung die mindestens ein kovalent gebundenes Kohlenstoffatom aufweist. Bei den erfindungsgemäß hergestellten Zusammensetzungen aus Metallverbindung und eingelagerter organischer Verbindung kann es sich um Einlagerungsverbindungen, Interkalationsverbindungen oder feste Lösungen handeln.

Bevorzugt handelt es sich um Einschlußverbindungen, Interkalationsverbindungen bzw. feste Lösungen eines Azobarbitursäure-Nickel-1:1-Komplexes der Struktur 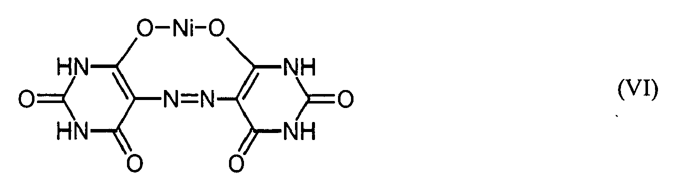 oder einer tautomeren Struktur dazu und mindestens einer anderen darin eingeschlossenen organischen Verbindung.

Besonders bevorzugt handelt es sich um Interkalationsverbindungen der vorstehend beschriebenen Metallverbindung der Formel (VI) mit Melamin in einem molaren Verhältnis von 1 : 2.

Im allgemeinen bilden die erfindungsgemäß hergestellten Metallverbindungen schichtförmigen Kristallgitter, bei dem die Bindung innerhalb einer Schicht im wesentlichen über Wasserstoffbrücken und/oder Metallionen erfolgt. Vorzugsweise handelt es sich dabei um Metallkomplexe, die ein Kristallgitter ausbilden, das aus im wesentlichen ebenen Schichten besteht.

Bevorzugt erfolgt die erfindungsgemäße Herstellung der Metallkomplexe der Azoverbindung der Formel (I) bzw. der Einlagerungsverbindungen davon in Gegenwart von Impfkristallen, die bevorzugt die gleiche chemische Struktur besitzen wie die nach dem erfindungsgemäßen Verfahren herzustellenden Metallverbindungen der Azoverbindung der Formel (I) bzw. die Einlagerungsverbindungen davon. Insbesondere werden, wenn das herzustellende Produkt eine Zusammensetzung aus einer Metallverbindung der Azoverbindung der Formel (I) und einer darin eingelagerten Verbindung ist, auch Impfkristalle einer solchen Einschlusszusammensetzung verwendet. Die Verwendung von Impfkristallen bei der Herstellung der Metallverbindungen oder der Einlagerungsverbindungen davon führt im allgemeinen zu höheren spezifischen Oberflächen nach BET. Obwohl die erfindungsgemäß durchgeführte mehrstufige Temperung im allgemeinen zu einer Verringerung der spezifischen Oberflächen nach BET führt, kann es im Rahmen der Erfindung gleichwohl vorteilhaft sein, die Herstellung der Metallverbindungen oder der Einlagerungsverbindungen davon in Gegenwart von Impfkristallen duchzuführen. Zum Einen führt die Anwendung der Impfkristalle zu einer Verbesserung der Reproduzierbarkeit der Herstellung, insbesondere beim Batch-Verfahren, wie es hier bevorzugt angewendet wird. Zum Anderen kann es aber auch sinnvoll sein, vor der Temperung von einem möglichst hohen Niveau der spezifischen Oberfläche der Metallverbindungen oder der Einlagerungsverbindungen davon auszugehen. Es hat sich überraschend herausgestellt, dass die physikalischen Eigenschaften der verwendeten Impfkristalle nicht notwendigerweise die physikalischen Eigenschaften der herzustellenden Metallverbindungen bestimmen. So werden beispielsweise Metallverbindungen mit hoher spezifischer Oberfläche nach BET auch dann erhalten, wenn die eingesetzten Impfkristalle eine vergleichweise geringe spezifische Oberfläche nach BET aufweisen. Bevorzugt erfolgt die Herstellung in Gegenwart von 1 ppm ― 10.000 ppm an Impfkristallen bezogen auf die theoretisch erhältliche Menge der herzustellenden Metallverbindung eines gegebenen Reaktionsansatzes, insbesondere von 10 ppm ― 5.000 ppm, ganz besonders bevorzugt von 50 ppm ― 3.000 ppm, insbesondere von 100 ppm ― 2.000 ppm. Ein besonders bevorzugtes erfindungsgemäßes Verfahren besteht darin, dass Impfkristalle durch Zurücklassen der gewünschten Menge Produkt aus einer Vorläuferpartie in den Reaktor eingebracht oder dort belassen werden. Das Zurücklassen der gewünschten Menge Produkt kann insbesondere bei Serienproduktionen ökonomisch vorteilhaft eingesetzt werden.

Je nach Ausgangsmaterial, dass in die Tempererungsstufen eingesetzt wird, können so nach dem erfindungsgemäßen Verfahren spezifische Oberflächen nach BET der Metallverbindungen der Azoverbindungen der Formel (I) bzw. der Zusammensetzung mit mindestens einer eingelagerten Verbindung davon von 60 bis 180 m²/g, insbesondere von 70 bis 160 m²/g, bevorzugt von 80 bis 140 m²/g und insbesondere von 90 bis 120 m²/g erhalten werden. Die spezifische Oberfläche wird nach DIN 66131 ermittelt: Bestimmung der spezifischen Oberfläche von Feststoffen durch Gasadsorption nach Brunauer, Emmett und Teller (B.E.T.).

Das erfindungsgemäße Verfahren wird bevorzugt chargenweise bzw. als sogenanntes Batch-Verfahren durchgeführt. Der Begriff "Batch-Verfahren" meint, wie dem Fachmann gut bekannt ist, ein diskontinuierliches Verfahren. D.h., die Herstellung der Metall-Verbindungen wird nicht kontinuierlich sondern ansatz- bzw. chargenweise durchgeführt. Nach Durchführung eines Reaktionsansatzes wird das Produkt isoliert. Im Gegensatz dazu werden beim kontinuierlichen Verfahren kontinuierlich Edukte zugeführt und Produkt abgeführt.

Im Rahmen dieser Anmeldung werden die Metallverbindungen der Azoverbindungen der Formel (I), die vorzugsweise wenigstens eine Verbindung eingelagert enthalten, auch als erfindungsgemäß hergestellte Pigmente bezeichnet.

Als Metallverbindungen kommen auch solche in Frage, bei denen eine metallhaltige Verbindung, z.B. ein Salz oder ein Metallkomplex in das Kristallgitter eines anderen Metallkomplexes z.B. des Nickelkomplexes eingebaut ist. In diesem Fall kann beispielsweise in Formel (VI) ein Teil des Metalls, z.B. des Nickels durch andere Metallionen ersetzt sein oder es können weitere Metallionen in eine mehr oder weniger starke Wechselwirkung mit der Metallverbindung, vorzugsweise einem Nickelkomplex treten.

Einschlussverbindungen, Interkalationsverbindungen und feste Lösungen von Metallkomplexen an sich sind aus der Literatur bekannt. Sie werden ebenso wie deren Herstellung beispielsweise in der EP 0 074 515, der EP 0 073 463 , der EP 0994163 sowie der EP 0994162 (dort Seite 5, Zeile 40 bis Seite 7, Zeile 58) beschrieben. Insoweit kann vollinhaltlich auf die Aufzählung der in Frage kommenden Verbindungen in diesen Druckschriften Bezug genommen werden.

Besonders bevorzugt werden als eingelagerte Verbindungen Melamin oder Melaminderivate, insbesondere solche der Formel (VII) 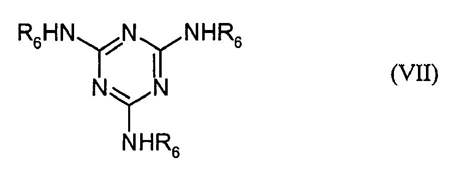 eingesetzt, worin
- R₆: für Wasserstoff oder C₁-C₄-Alkyl, das gegebenenfalls mit OH-Gruppen substituiert ist, steht,
ganz besonders bevorzugt, worin
- R₆: für Wasserstoff steht.

Die Menge an eingelagerter Substanz, die in das Kristallgitter des Metallkomplexes eingelagert werden kann, liegt in der Regel bei 5 % bis 200 Gew.-%, bezogen auf die Menge an Metallverbindung. Bevorzugt eingelagert werden 10 bis 100 Gew.-%. Es handelt sich hierbei um die Menge an eingelagerter Substanz, die durch geeignete Lösungsmittel nicht auswaschbar ist und die sich aus der Elementaranalyse ergibt. Naturgemäß kann auch mehr oder weniger als die genannte Menge an Substanz zugesetzt werden, wobei man gegebenenfalls darauf verzichten kann, einen Überschuß auszuwaschen. Bevorzugt sind Mengen von 10 bis 150 Gew.-% bezogen auf die Menge an Metallverbindung.

Die Herstellung der Metallverbindungen bzw. der Einlagerungsverbindungen davon erfolgt beispielsweise wie in EP 0 074 515, EP 0 073 463, EP 0994163 sowie der EP 0994162 beschrieben. Nach der Synthese der Azoverbindung wird im allgemeinen in Gegenwart der zu interkalierenden Verbindung mit einem Metallsalz komplexiert. Im Falle der technisch interessanten Interkalationsverbindungen der Metallkomplexe der zwei- und dreiwertigen Metalle, besonders der technisch und wirtschaftlichen wichtigen Interkalationsverbindung des Azobarbitursäure-Nickel-Komplexes, erfolgen Komplexierung und Interkalation sowie auch die nachfolgende Isolierung zweckmäßig im sauren pH-Bereich.

Als Metallsalz kommen vorzugsweise wasserlösliche Metallsalze der oben genannten Metalle in Frage, insbesondere Chloride, Bromide, Acetate, Nitrate usw. bevorzugt des Nickels in Frage. Bevorzugt eingesetzte Metallsalze besitzen eine Wasserlöslichkeit von mehr als 20 g/l, insbesondere mehr als 50 g/l bei 20 °C.

Man kann auch Mischungen dieser Salze, die verschiedene der genannten Metalle enthalten können, verwenden. Die Verwendung von solchen Salzmischungen empfiehlt sich insbesondere für die Erzielung von Zwischentönen der farbigen Endprodukte.

In einer bevorzugten Ausführungsform wird das erfindungsgemäße Verfahren als Batchverfahren in einem Reaktor, beispielsweise in einem Rührkesselreaktor, bevorzugt unter Anwendung einer Umpumpung durchgeführt. "Umpumpung" bedeutet dabei, dass Mittel vorgesehen sind, mit denen während der Herstellung dem Reaktor Inhalt entnommen und wiederzugeführt werden kann. Eine bevorzugte Ausgestaltung einer solchen Umpumpung besteht darin, dass der verwendete Reaktor, insbesondere ein Rührkessel ein vorzugsweise außerhalb des Reaktors liegendes Rohrleitungssystem aufweist. Das Rohrleitungssystem ist mit dem Reaktor bzw. Reaktorinhalt an mindestens zwei verschiedenen Stellen verbunden. Das Rohrleitungssystem weist Mittel auf, mit denen dem Reaktor an einer oder mehreren Stellen Reaktorinhalt entnommen werden kann und nach Passieren des Rohrleitungssystems an einer oder mehreren anderen Stellen wieder zugeführt werden kann. Derartige Mittel sind insbesondere Pumpen. Das erfindungsgemäß verwendete Umpumpungssystem weist bevorzugt Dosiereinrichtungen auf, die es ermöglichen, in das außerhalb des Reaktors liegende Rohrleitungssystem Reaktionspartner, beispielsweise Edukte, Lösungen von Edukten, Säuren, Basen etc. zu geben.

Ein besonders bevorzugtes erfindungsgemäßes Verfahren besteht darin, Säuren und Basen nicht direkt in den Reaktor sondern in das Umpumpungssystem zu dosieren. Ein besonders bevorzugtes erfindungsgemäßes Verfahren besteht darin, Reaktanden, Säuren und/oder Laugen bzw. Basen so zu dosieren, dass die Dosierzeit dem 0,2 fachen - 5 fachen eines theoretischen Gesamtumpumpcyclus, insbesondere dem 1 fachen - 2 fachen entspricht. Der theoretische Gesamtumpumpcyclus bedeutet den Zeitraum, innerhalb dessen das Volumen des Reaktorinhalts das Umpumpungssystem einmal passiert hat.

Es wird angenommen, dass die Umpumpung einen Bereich schafft, der eine vergleichsweise hohe Strömungsgeschwindigkeit aufweist. Diese Strömungsgeschwindigkeit liegt im allgemeinen höher als die Strömungsgeschwindigkeit im Rührkesselreaktor an Stellen geringer Rührwirkung, wie z.B. im Bereich oberhalb des obersten Rührblatts. Insbesondere bei Zudosierung im Bereich des Umpumpungssystems können durch die dort herrschende hohe Strömungsgeschwindigkeit lokale Konzentrationsspitzen vermieden werden. Weiterhin wird insgesamt eine bessere Durchmischung des Reaktorinhalts sichergestellt. Überraschend wird durch das erfindungsgemäße Verfahren ein Produkt erhalten, das eine noch höhere spezifische Oberfläche aufweist als ein Produkt, das ohne Umpumpungsverfahren hergestellt wurde. Weiterhin führt die Anwendung der Umpumpung zu einer zusätzlichen Verringerung der Schwankungen der Produktqualität. Es können mehrere Umpumpsysteme parallel eingesetzt werden.

Besonders bevorzugt werden als Pigmente solche in das erfindungsgemäße Verfahren eingesetzt, die unmittelbar durch Umsetzung von Azoverbindungen der Formel (I) mit Metallsalzen, vorzugsweise solchen mit einer Wasserlöslichkeit von mehr als 20, insbesondere mehr als 50 g/l bei 20°C und anschließend durch Umsetzung mit der zu interkalierenden Verbindung erhalten werden.

Die ungetemperten Pigmente, im folgenden Edukte genannt, werden vorzugsweise wie folgt so erhalten, daß die Umsetzung mit der Metallverbindung bei einen pH ≤ 2 erfolgt. Die anschließende Interkalation erfolgt vorzugsweise bei einem pH von 1 bis 7. Sofern die Interkalation bei pH < 4 durchgeführt wird, ist es bevorzugt, im Anschluß den pH auf größer 4,5, vorzugsweise 4,5 bis 7 anzuheben.

Zur Einstellung des pH-Wertes werden vorzugsweise anorganische oder organische Säuren und Basen eingesetzt

Als bevorzugte Säuren werden HCl, H₃PO₄, H₂SO₄, JH, HBr, Essigsäure und/oder Ameisensäure eingesetzt.

Als bevorzugte Basen werden LiOH, KOH, NaOH, Ca(OH)₂, NH₃, Ethanolamin, Diethanolamin, Triethanolamin und/oder Dimethylethanolamin eingesetzt.

Diese Eduktsuspension kann nun einerseits filtriert werden und das verbleibende Edukt vorzugsweise mit Wasser, insbesondere heißem Wasser gewaschen werden, um nicht interkalierte Anteile, Salze sowie sonstige Verunreinigungen abzutrennen.

Das so erhaltene Edukt kann zwischenisoliert und gegebenenfalls getrocknet werden.

Vorzugsweise werden jedoch direkt aus der Synthese im Eintopfverfahren erhaltene Metallverbindungen oder Einlagerungsverbindungen ohne Zwischenisolierung der mehrstufigen Temperung unterworfen, vorzugsweise in wenigstens zwei pH-Stufen bei pH-Werten zwischen 0 bis 4 und Temperaturen von 80 bis 125°C, vorzugsweise in einem Eintopfverfahren. (Temperaturen von mehr als 100°C implizieren in wässrigen Medien, wie dem Fachmann bekannt ist, Drücke die oberhalb des Normaldrucks liegen).

Die nach dem erfindungsgemäßen Verfahren getemperte Suspension, enthaltend das erfindungsgemäße Pigment, wird vorzugsweise nach der Temperung erneut auf einen pH von 4,5 bis 7 gestellt. Danach wird vorzugsweise filtriert. Der so erhaltene Presskuchen kann gegebenenfalls nach Waschen mit Wasser getrocknet werden.

Dabei kommen übliche Trocknungsverfahren wie die Schaufeltrocknung usw. in Frage. Mit derartigen Trocknungsverfahren und anschließender Mahlung des Pigments auf übliche Weise werden pulverförmige Pigmente erhalten.

Bevorzugt wird der Presskuchen als wässrige Slurry sprühgetrocknet. Besonders bevorzugt erfolgt dies durch Versprühen eines Slurrys, der zur Erhöhung des Feststoffanteils Ammoniak enthält. Der zu versprühende Slurry besitzt vorzugsweise einen Feststoffanteil von 10 bis 40 Gew.-%, insbesondere 15 bis 30 Gew.-%.

Weiterhin können dem Slurry Viskositäts-erniedrigenden Zusätze wie Carbonsäure und Sulfonsäureamide in einer Menge von bis zu 10 Gew.-%, bezogen auf den Slurry zugesetzt werden.

Die Erfindung betrifft weiterhin ein Verfahren zur Herstellung von Pigmentpräparationen, worin wenigstens eine erfindungsgemäß hergestellte Metallverbindung oder eine Einlagerungsverbindung davon und wenigstens ein Dispergiermittel vermischt werden. Diese Pigmentpräparationen dienen vorzugsweise der Einarbeitung in wässrige Systeme.

Bezüglich geeigneter Dispergiermittel kann auf den eingangs erwähnten Stand der Technik verwiesen werden, insbesondere die EP-A1-0994164, Seite 8, Zeile 56 Seite 11, Zeile 23, deren Offenbarung zum Gegenstand dieser Anmeldung gehören. Besonders bevorzugt enthält die Pigment-Präparation mehr als 90 insbesondere mehr als 95, vorzugsweise mehr als 97 Gew.-% an Pigment (erfindungsgemäß hergestellte Metallverbindung + gegebenenfalls darin eingelagerte Verbindung(en)) und Dispergiermittel.

Die nach dem erfindungsgemäßen mehrstufigen Temperungsverfahren hergestellten Pigmente weisen überraschenderweise vorteilhafte Eigenschaften auf. Die Pigmente zeigen vorteilhafte Farbstärke, Brillanz, Einheitlichkeit, Dispergierbarkeit und/oder wirtschaftliche Herstellbarkeit.

Die erfindungsgemäß hergestellten Metallverbindungen oder Einlagerungsverbindungen davon oder Pigmentpräparationen eignen sich überdies hervorragend für alle Pigmentanwendungszwecke.

Zum Beispiel eignen sie sich zum Pigmentieren von Lacken aller Art für die Herstellung von Druckfarben, Leimfarben oder Binderfarben, für die Massefärbung von synthetischen, halbsynthetischen oder natürlichen makromolekularen Stoffen, wie z.B. Polyvinylchlorid, Polystyrol, Polyamid, Polyethylen oder Polypropylen. Sie können auch für die Spinnfärbung von natürlichen, regenerierten oder künstlichen Fasern, wie z.B. Cellulose-, Polyester-, Polycarbonat-, Polyacrylnitril- oder Polyamidfasern, sowie zum Bedrucken von Textilien und Papier verwendet werden. Aus diesen Pigmenten können feinteilige, stabile, wässrige Pigmentierungen von Dispersions- und Anstrichfarben, die für die Papierfärbung, für den Pigmentdruck von Textilien, für den Laminatdruck oder für die Spinnfärbung von Viskose brauchbar sind, durch Mahlen oder Kneten in Gegenwart von nichtionogenen, anionischen oder kationischen Tensiden hergestellt werden. Die nach dem erfindungsgemäßen Verfahren hergestellten Pigmente eignen sich hervorragend für Ink Jet Anwendungen und aufgrund ihrer vergleichsweise hohen spezifischen Oberfläche nach BET für Farbfilter für Flüssigkristallanzeigen.

### Beispiele

### Herstellungs-Beispiel 1

190 g wasserfeuchte Paste von Diazobarbitursäure mit einem Trockengehalt von 81 %, entsprechend 154g trocken, werden in 3000 g Wasser mit einem Laborrührer verrührt. Danach wird indirekt auf 80°C erwärmt und bei dieser Temperatur 134 g Barbitursäure eingetragen. Nach ca. 30 minütigem Nachrühren wird mit 30%iger Kaliumhydroxidlösung auf pH 5,0 gestellt. Anschließend 2 Stunden bei pH 5,0 und 80°C nachgerührt. Danach wird mit Wasser auf 5400 g verdünnt. Anschließend wird indirekt auf 90°C erwärmt und bei dieser Temperatur 252 g Melamin eingetragen. Danach werden 575 g 22,5%iger Nickelchloridlösung zugetropft. 90 Minuten wird nachgerührt, um eine möglichst vollständig Umsetzung zu erreichen. Dann wird mit 30%iger Kaliumhydroxidlösung auf pH 5,0 gestellt. Danach wird die ungetemperte Suspension auf einer Saugnutsche isoliert, elektrolytfrei gewaschen, bei 80°C im Vakuumtrockenschrank getrocknet und in einer üblichen Labormühle ca. 2 Minuten gemahlen.

### Vergleichs-Beispiel 1 (einstufige Temperung)

Ca. 6275 g ungetemperte Suspension einer nach Herstellungs-Beispiel 1 hergestellten Melamin-Interkalationsverbindung des Azobarbitursäure-Nickel-Komplexes mit einem Pigmentgehalt von 9,4 %, entsprechend 591 g trocken, werden mit destilliertem Wasser auf einen Pigmentgehalt von z. B. 7,5 % verdünnt. Dann wird mit Salzsäure direkt auf pH 1,5 gestellt und eine Temperatur von 98°C für 12 h gehalten. Danach wird auf 95°C abgekühlt und zum Isolieren mit Kaliumhydroxidlösung auf pH 5,0 eingestellt.

Anschließend wird auf einer Saugnutsche isoliert, elektrolytfrei gewaschen, bei 80°C im Vakuumtrockenschrank getrocknet und in einer üblichen Labormühle ca. 2 Minuten gemahlen.

### Erfindungs-Beispiel 1 und folgende

Ca. 6275 g ungetemperte Suspension einer nach Herstellungs-Beispiel 1 hergestellten Melamin-Interkalationsverbindung des Azobarbitursäure-Nickel-Komplexes mit einem Pigmentgehalt von 9,4 %, entsprechend 591 g trocken, werden mit destilliertem Wasser auf einen Pigmentgehalt von z. B. 7,5 % verdünnt. Dann wird mit Salzsäure auf pH 3 gestellt und eine Temperatur von 98°C für 30min gehalten. Dann wird auf pH 1,5 gestellt die Temperatur für 11,5 h gehalten. Danach wird auf 95°C abgekühlt und zum Isolieren mit Kaliumhydroxidlösung auf pH 5,0 eingestellt.

Anschließend wird auf einer Saugnutsche isoliert, elektrolytfrei gewaschen, bei 80°C im Vakuumtrockenschrank getrocknet und in einer üblichen Labormühle ca. 2 Minuten gemahlen.

Mit dem Vergleichsbeispiel 1 und den Erfindungs-Beispielen wurden mit den in Tabelle 1 aufgeführten Temperbedingungen die dort aufgeführten Eigenschaften erhalten.

Jeweils 4g des zu prüfenden Pigmentes wurden mit 396g einer kommerziellen Weißpaste, beispielsweise Ready Nova 70 der Firma Nordsjö (Akzo Nobel) und 400 ml Glasperlen vom Durchmesser 2 mm in einer Süßmeier Perlmühle unter Kühlen 30 min gemahlen. Die Pasten wurden mit einer Spiralrakel (25 µm) auf Rakelpapier aufgetragen und farbmetrisch mit dem Farbmessgerät Color Guide 450 der Firma Gardner vermessen.

Das Prinzip der Farbmessung wird beispielsweise in der Bayer Farben Revue, Sonderheft 3/2 D, Farbmessung 1986 beschrieben.

Der Nullversuch (Vergleichsbeispiel 1) hat definitionsgemäß die Farbstärke 100%.

Bevorzugt ist eine möglichst hohe Farbstärke; insbesondere eine möglichst hohe Farbstärke bei vergleichsweise niedriger BET.

Der Quotient Farbstärke _{erfindungsgemäß}/Farbstärke _{Nullversuch} ist vorzugsweise > 1, insbesondere > 1,05, ganz besonders bevorzugt > 1,1.

Farbstärke Nullversuch bedeutet dabei die Farbstärke der Metallverbindungen bzw. der Einlagerungsverbindungen davon, die maximal einem Temperschritt unterworfen wurden.

**Tabelle 1: Temperung**

| **Beispiel/Vergleichsbeispiel** | **Temperatur I (°C)** | **pH Stufe I** | **Zeit (h)** | **Temperatur H (°C)** | **pH Stufe H** | **Zeit (h)** | **Farbstärke** |
|---|---|---|---|---|---|---|---|
| 1* | 98 | - | - | 98 | 1,5 | 12 | 100 |
| 1 | 98 | 3 | 0,5 | 98 | 1,5 | 11,5 | 105 |
| 2 | 98 | 3 | 1 | 98 | 1,5 | 11 | 107 |
| 3 | 98 | 3 | 2 | 98 | 1,5 | 10 | 110 |
| 4 | 98 | 3 | 4 | 98 | 1,5 | 8 | 111 |
| 5 | 98 | 3 | 6 | 103 | 1,5 | 6 | 112 |

| | | | | | | | |
|---|---|---|---|---|---|---|---|
| *) Vergleichs-Beispiel 1 | | | | | | | |

### Vergleichs-Beispiel 2 (einstufige Temperung)

a) In einem 20m³ Reaktor mit Mantelheiz-/kühl-System, Rührer, Stromstörer und Umpumpsystem werden 6.000 Liter 80°C heißes Wasser bei einer Rührgeschwindigkeit von 20 U/min vorgelegt. 380 kg wasserfeuchte Paste von Diazobarbitursäure mit einem Trockengehalt von 81 %, entsprechend 308 kg trocken, werden eingetragen.
   Die Temperatur wird auf 80°C gehalten und bei dieser Temperatur 268 kg Barbitursäure eingetragen. Es wird mit einer Umpumpung gearbeitet, die auf 15m³/h eingestellt ist. Nach 1 Stunde Umpumpung wird mit 3 0%iger Kaliumhydroxidlösung innerhalb 30min auf pH 5,0 gestellt, wobei die Kaliumhydroxidlösung in die Umpumpung dosiert wird. Anschließend wird 2 Stunden bei pH 5,0 und 80°C unter Umpumpen nachgerührt. Danach wird mit Wasser auf 15.000 Liter verdünnt. Anschließend wird auf 90°C erwärmt und bei dieser Temperatur 500 kg Melamin eingetragen. Die Umpumpung wird auf 30m³/h gestellt. Danach werden 1.150 kg 22,5 %iger Nickelchloridlösung über den Umpumpkreislauf innerhalb 30min eindosiert. 90 Minuten wird unter Umpumpen nachgerührt, um eine möglichst vollständige Umsetzung zu erreichen. Dann wird mit 30%iger Kaliumhydroxidlösung innerhalb 30min auf pH 5,0 gestellt, wobei die Kaliumhydroxidlösung in die Umpumpung eindosiert wird.
b) Im nicht-erfindungsgemäßen einstufigen Temperungsverfahren werden zu dieser Suspension 125 kg 30 %ige Salzsäure direkt als Festmenge (in einer Portion) zugegeben und eine Temperatur von 98°C für 12 Stunden gehalten. Danach wird mit 30 %iger Kaliumhydroxidlösung innerhalb 30min auf pH 5,0 gestellt, wobei die Kaliumhydroxidlösung in den Umpumpkreislauf eindosiert und die Temperatur auf 80°C geregelt wird.
   Der anbackungsfreie Reaktor lässt sich sehr leicht praktisch vollständig entleeren. Die homogene Pigment-Slurry wird auf einer Filterpresse isoliert, elektrolytfrei gewaschen und bei 80°C getrocknet.

### Erfindungs-Beispiel 6

In der ersten Temperungsstufe wird in die Suspension erhalten nach a) von Vergleichsbeispiel 2 bei 90°C über eine Dosierung von ca. 50 kg 30 %iger Salzsäure in den Umpumpkreislauf innerhalb 30 min pH 3,0 eingestellt. In der zweiten Temperungsstufe werden 75 kg 30 %ige Salzsäure direkt als Festmenge zugegeben und eine Temperatur von 98°C für 11,5 Stunden gehalten. Danach wird mit 30 %iger Kaliumhydroxidlösung innerhalb 30min auf pH 5,0 gestellt, wobei die Kaliumhydroxidlösung in den Umpumpkreislauf eindosiert und die Temperatur auf 80°C geregelt wird.

Der anbackungsfreie Reaktor lässt sich sehr leicht praktisch vollständig entleeren. Die homogene Pigment-Slurry wird auf einer Filterpresse isoliert, elektrolytfrei gewaschen und bei 80°C getrocknet.

### Erfindungs-Beispiel 7

In der ersten Temperungsstufe wird in die Suspension erhalten nach a) von Vergleichsbeispiel 2 bei 90°C über eine Dosierung von ca. 50 kg 30%iger Salzsäure in den Umpumpkreislauf innerhalb 30 min pH 3,0 eingestellt und 30min nachgerührt. In der zweiten Temperungsstufe werden 75 kg 30%ige Salzsäure direkt als Festmenge zugegeben und eine Temperatur von 98°C für 11 Stunden gehalten. Danach wird mit 30%iger Kaliumhydroxidlösung innerhalb 30min auf pH 5,0 gestellt, wobei die Kaliumhydroxidlösung in den Umpumpkreislauf eindosiert und die Temperatur auf 80°C geregelt wird.

Der anbackungsfreie Reaktor lässt sich sehr leicht praktisch vollständig entleeren. Die homogene Pigment-Slurry wird auf einer Filterpresse isoliert, elektrolytfrei gewaschen und bei 80°C getrocknet.

### Erfindungs-Beispiel 8

In der ersten Temperungsstufe wird in die Suspension erhalten nach a) von Vergleichsbeispiel 2 bei 90°C über eine Dosierung von ca. 50 kg 30%iger Salzsäure in den Umpumpkreislauf innerhalb 30 min pH 3,0 eingestellt und 30min nachgerührt. In der zweiten Temperungsstufe werden direkt 80 kg Kaliumhydrogensulfat zugegeben und eine Temperatur von 98°C für 11 Stunden gehalten. Danach wird mit 30%iger Kaliumhydroxidlösung innerhalb 30min auf pH 5,0 gestellt, wobei die Kaliumhydroxidlösung in den Umpumpkreislauf eindosiert und die Temperatur auf 80°C geregelt wird.

Der anbackungsfreie Reaktor lässt sich sehr leicht praktisch vollständig entleeren. Die homogene Pigment-Slurry wird auf einer Filterpresse isoliert, elektrolytfrei gewaschen und bei 80°C getrocknet.

Proben aus dem Vergleichsbeispiel 2 und den Erfindungs-Beispielen 6, 7 und 8 wurden jeweils in einer üblichen Labormühle ca. 2 Minuten gemahlen und der nachstehenden Prüfungen unterzogen.

Jeweils 4g des zu prüfenden Pigmentes wurden mit 396g einer kommerziellen Weißpaste, beispielsweise Ready Nova^{®} 70 der Firma Nordsjö (Akzo Nobel) und 400 ml Glasperlen vom Durchmesser 2 mm in einer Süßmeier Perlmühle unter Kühlen 30 min gemahlen. Die Pasten wurden mit einer Spiralrakel (25 µm) auf Rakelpapier aufgetragen und farbmetrisch mit dem Farbmessgerät Color Guide 450 der Firma Gardner vermessen.

Das Prinzip der Farbmessung wird beispielsweise in der Bayer Farben Revue, Sonderheft 3/2 D, Farbmessung 1986 beschrieben.

Der Nullversuch (Vergleichsbeispiel 2) hat definitionsgemäß die Farbstärke 100%.

| | |
|---|---|
| Vergleichsbeispiel 2 | Farbstärke: 100 |
| Erfindungsbeispiel 6 | Farbstärke: 105 |
| Erfindungsbeispiel 7 | Farbstärke: 108 |
| Erfindungsbeispiel 8 | Farbstärke: 106 |

## Patentansprüche

1. Verfahren zur Herstellung von Metall-Verbindungen einer Azo-Verbindung der Formel (I) 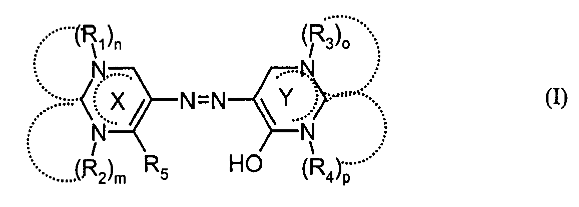 oder tautomere Strukturen davon,
worin die mit
X und Y bezeichneten Ringe je einen oder zwei Substituenten aus der Reihe =O, =S, =NR₇, -NR₆R₇, -OR₆, -SR₆, -COOR₆, -CN, -CONR₆R₇, -SO₂R₈, 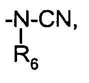 Alkyl, Cycloalkyl, Aryl, Aralkyl tragen können,
wobei die Summe der endo- und exocyclischen Doppelbindungen für jeden der Ringe X und Y drei ist,
worin
R₆ Wasserstoff, Alkyl, Cycloalkyl, Aryl oder Aralkyl ist,
R₇ Wasserstoff, Cyano, Alkyl, Cycloalkyl, Aryl, Aralkyl oder Acyl ist und
R₈ Alkyl, Cycloalkyl, Aryl oder Aralkyl ist,
R₁, R₂, R₃, R₄ für Wasserstoff, Alkyl, Cycloalkyl, Aryl oder Aralkyl stehen und weiterhin, wie in Formel (I) durch die unterbrochenen Linien angedeutet wird, 5- oder 6-gliedrige Ringe ausbilden können, an die weitere Ringe ankondensiert sein können,
R₅ -OH, -NR₆R₇, Alkyl, Cycloalkyl, Aryl oder Aralkyl bedeutet,
worin R₆ und R₇ wie oben definiert sind,
wobei in den für R₁ bis R₈ genannten Substituenten, die CH-Gruppen enthalten, die Wasserstoffatome in den CH-Gruppen substituiert sein können,
und m, n, o, p 1 (eins), oder für den Fall, daß von den Ring-Stickstoffatomen, an denen die entsprechenden Substituenten R₁ bis R₄ sitzen, Doppelbindungen ausgehen, wie in Formel (I) durch die punktierten Linien angedeutet wird, auch 0 (null) bedeuten können,
und die gegebenenfalls eine Verbindung eingelagert enthalten,
**dadurch gekennzeichnet, dass** die Metallverbindung oder eine Einlagerungsverbindung davon in wenigstens zwei pH-Stufen getempert wird.

2. Verfahren gemäß Anspruch 1, **dadurch gekennzeichnet, dass** in der Verbindung der Formel (I) der mit X **gekennzeichnet**e Ring für einen Ring der Formel 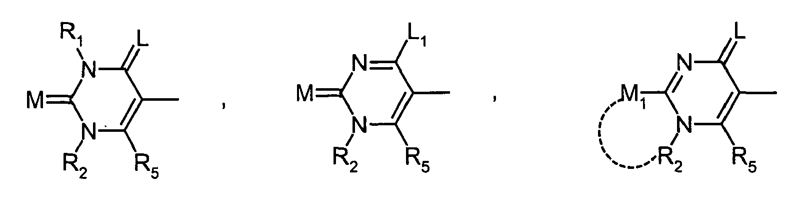 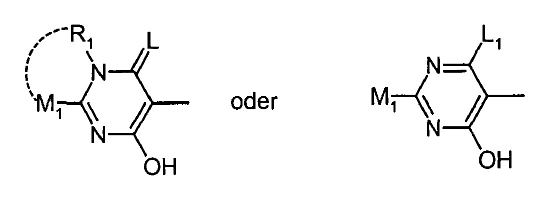 steht, in denen
L und M unabhängig voneinander für =O, =S oder =NR₆ stehen,
L₁ Wasserstoff, -OR₆, -SR₆, -NR₆R₇, -COOR₆, -CONR₆R₇, -CN, Alkyl, Cycloalkyl, Aryl, Aralkyl ist und
M₁ -OR₆, -SR₆, -NR₆R₇, -COOR₆, -CONR₆R₇, -CN, -SO₂R₈, 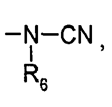 Alkyl, Cycloalkyl, Aryl oder Aralkyl bezeichnen,
oder die Substituenten M₁ und R₁ oder M₁ und R₂ einen 5- oder 6-gliedrigen Ring ausbilden können, und
R₁, R₂, R₅, R₆, R₇ und R₈ die in Anspruch 1 angegebenen Bedeutungen besitzen.

3. Verfahren gemäß Anspruch 1, **dadurch gekennzeichnet, dass** die Azoverbindung der Formel (I) in Form ihrer freien Säure der Formel (II) oder (III) oder einer dazu tautomeren Form entspricht 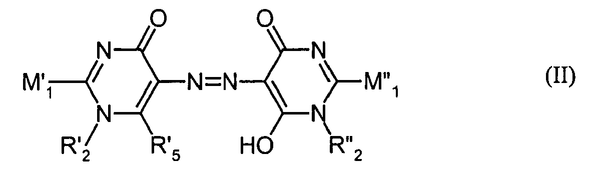 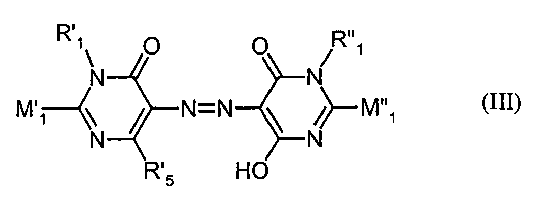 in denen
R'₅ -OH oder -NH₂ bezeichnet,
R'₁, R"₁, R'₂ und R"₂ jeweils für Wasserstoff stehen und
M'₁ und M"₁ unabhängig voneinander für Wasserstoff, -OH, -NH₂, -NHCN, Arylamino oder Acylamino stehen.

4. Verfahren gemäß Anspruch 1, **dadurch gekennzeichnet, dass** die Azoverbindung der Formel (I) in Form ihrer freien Säure der Formel (V) oder einer tautomeren Form davon entspricht

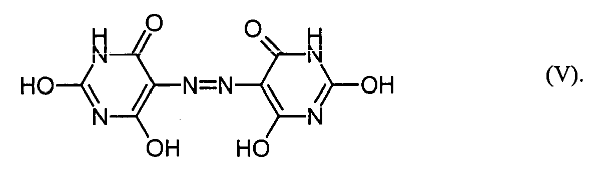

5. Verfahren gemäß einem der Ansprüche 1 bis 4, **dadurch gekennzeichnet, dass** die Metall-verbindungen der Azoverbindung der Formel (I) Salze bzw. Komplexverbindungen der Mono-, Di-, Tri- und Tetraanionen der Azoverbindung der Formel (I) mit einem oder mehreren Metallen sind, die aus der Gruppe ausgewählt werden, die besteht aus: Li, Na, K, Mg, Ca, Sr, Ba, Al, Sn, Pb, Sc, Ti, V, Cr, Mn, Fe, Co, Ni, Cu, Zn, Y, Zr, Nb, Mo, Cd, Hf, Ta, W, La, Ce, Pr und Nd.

6. Verfahren gemäß einem der Ansprüche 1 bis 5, **dadurch gekennzeichnet, dass** als Metallverbindung ein Ni-Salz bzw. ein Ni-Komplex der Azoverbindung der Formel (I) eingesetzt wird.

7. Verfahren zur Herstellung von Metallverbindungen gemäß einem der Ansprüche 1 bis 6, **dadurch gekennzeichnet, dass** sie eine cyclische oder acyclische organische Verbindung, insbesondere Melamin eingelagert enthalten.

8. Verfahren zur Herstellung der Metallverbindungen oder der Einlagerungsverbindungen davon gemäß einem der Ansprüche 1 bis 7, **dadurch gekennzeichnet, dass** die Temperungsstufen in einer Dispersion mit einem Feststoffgehalt zwischen 4 - 15 Gew.-%, ganz besonders bevorzugt zwischen 6 - 10 Gew.-% durchgeführt werden.

9. Verfahren zur Herstellung der Metallverbindungen oder der Einlagerungsverbindungen davon gemäß einem der Ansprüche 1 bis 8, **dadurch gekennzeichnet, dass** die Temperungsstufen bei Temperaturen zwischen 90°C und 120°C, insbesondere zwischen 95°C und 110°C durchgeführt werden.

10. Verfahren zur Herstellung der Metallverbindungen oder der Einlagerungsverbindungen davon gemäß einem der Ansprüche 1 bis 9, **dadurch gekennzeichnet, dass** der pH-Wert von wenigstens einer Temperungsstufe zwischen 2 und 4, insbesondere zwischen 2,5 und 3,5 liegt und der pH-Wert von einer zweiten Temperungsstufe zwischen 0 und 3, besonders bevorzugt zwischen 1 und 2,5 liegt und sich vorzugsweise die pH-Werte dieser zwei Temperungsstufen um 0,5 bis 3 Einheiten, vorzugsweise um 1 bis 2 Einheiten unterscheiden.

11. Verfahren zur Herstellung der Metallverbindungen oder der Einlagerungsverbindungen davon gemäß einem der Ansprüche 1 bis 10, **dadurch gekennzeichnet, dass** wenigstens eine, bevorzugt wenigstens zwei Temperungsstufe zwischen 0,25h und 24h dauern, insbesondere jeweils zwischen 1h und 12h, ganz besonders bevorzugt jeweils zwischen 2h und 8h.

12. Verfahren zur Herstellung der Metallverbindungen oder der Einlagerungsverbindungen davon gemäß einem der Ansprüche 1 bis 11, **dadurch gekennzeichnet, dass** eine spezifische Oberfläche nach BET des Pigmentes zwischen 60 und 180 m²/g eingestellt wird.

13. Verfahren zur Herstellung der Metallverbindungen gemäß einem der Ansprüche 1 bis 12 oder der Einlagerungsverbindungen davon, **dadurch gekennzeichnet, dass** die nach dem Verfahren gemäß einem der Ansprüche 1 bis 12 hergestellten Metallverbindungen oder die Einlagerungsverbindungen davon als wässrige Slurry sprühgetrocknet werden.

14. Verfahren zur Herstellung von Farbfiltern in Flüssigkristallanzeigen, dass die Verwendung der Metallverbindungen, hergestellt nach einem Verfahren gemäß einem der Ansprüche 1 bis 13 oder der Einlagerungsverbindungen davon umfasst.

15. Verfahren zur Herstellung von Pigmentpräparationen, worin wenigstens eine Metallverbindung oder eine Einlagerungsverbindung davon, hergestellt nach einem Verfahren nach einem der Ansprüche 1 bis 13 und wenigstens ein Dispergiermittel vermischt werden.

16. Verwendung der Metallverbindungen, hergestellt nach einem Verfahren gemäß einem der Ansprüche 1 bis 13 oder der Einlagerungsverbindungen davon oder der Pigmentpräparationen hergestellt nach Anspruch 15 als Pigment.

17. Verwendung der Metallverbindungen, hergestellt nach einem Verfahren gemäß einem der Ansprüche 1 bis 13 oder der Einlagerungsverbindungen davon, oder der Pigmentpräparationen hergestellt nach Anspruch 15 zur Herstellung von Druckfarben, Leimfarben oder Binderfarben, für die Massefärbung von synthetischen, halbsynthetischen oder natürlichen makromolekularen Stoffen, insbesondere Polyvinylchlorid, Polystyrol, Polyamid, Polyethylen oder Polypropylen, sowie für die Spinnfärbung von natürlichen, regenerierten oder künstlichen Fasern, wie z.B. Cellulose-, Polyester-, Polycarbonat-, Polyacrylniril- oder Polyamidfasern, sowie zum Bedrucken von Textilien und Papier.

18. Verwendung der Metallverbindungen, hergestellt nach einem Verfahren gemäß einem der Ansprüche 1 bis 13, oder der Einlagerungsverbindungen davon oder der Pigmentpräparationen hergestellt nach Anspruch 17 als Pigment für Laminate, als Pigment für Farbfilter in Flüssigkristallanzeigen, als Pigment für den Ink Jet Druck.
